# EUROPEAN PATENT SPECIFICATION

(11) **EP 4 213 308 B1**
(45) Date of publication and mention of the grant of the patent: **25.06.2025**
(21) Application number: 22207644.0
(22) Date of filing: 15.11.2022
(51) Int. Cl.: H01R 12/59, H01R 12/63, H01R 4/06, H01R 11/12

(54) **CONNECTOR**
VERBINDER
CONNECTEUR

(30) Priority: 12.01.2022 JP 2022002923
(43) Date of publication of application: 19.07.2023
(73) Proprietor: Japan Aviation Electronics Industry, Ltd., Tokyo 150-0043 (JP)
(72) Inventor: KINOSHITA, Takumi, Tokyo, 150-0043 (JP); MATSUNAGA, Akihiro, Tokyo, 150-0043 (JP); ASHIBU, Kenta, Tokyo, 150-0043 (JP)
(74) Representative: Qip Patent & Recht Dr. Kuehn & Partner mbB

(56) References cited:
- EP-A1- 3 361 575
- EP-A1- 3 435 490
- EP-A1- 3 800 739
- WO-A1-2020/018009
- DE-T5- 112017 005 137
- JP-A- 2014 203 815
- JP-U- S5 613 369
- US-B1- 6 310 407

## Description

### BACKGROUND OF THE INVENTION

The present invention relates to a connector for connecting an electric wire forming a a conductive connection object to a flexible conductor of a sheet-like conductive member.

In recent years, attention has been drawn to so-called smart clothes that can obtain user's biological data such as the heart rate and the body temperature only by being worn by the user. Such a smart cloth is equipped with an electrode disposed at a measurement position and formed of a flexible conductor, and when a wearable device serving as a measurement device is electrically connected to the electrode, it is possible to send biological data to the wearable device.

Connection between the electrode and the wearable device can be established by using, for example, a connector to be connected to the flexible conductor.

As a connector of this type, for example, JP 2018-129244 A discloses a connector shown in FIG. 26. This connector includes a housing 2 and a base member 3 that are separately disposed on opposite sides across a flexible substrate 1 to sandwich the flexible substrate 1. A tubular portion 4A of a contact 4 is passed through a contact through-hole 2A of the housing 2, and a flange 4B of the contact 4 is sandwiched between the housing 2 and a flexible conductor 1A exposed on a surface of the flexible substrate 1.

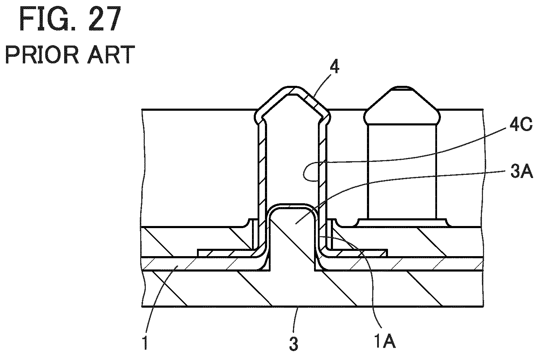

In this state, by pushing the base member 3 toward the housing 2, as shown in FIG. 27, a projection 3A of the base member 3 is inserted into a projection accommodating portion 4C of the contact 4 with the flexible substrate 1 being sandwiched therebetween, and an inner surface of the projection accommodating portion 4C makes contact with the flexible conductor 1A with a predetermined contact force, whereby the contact 4 is electrically connected to the flexible conductor 1A.

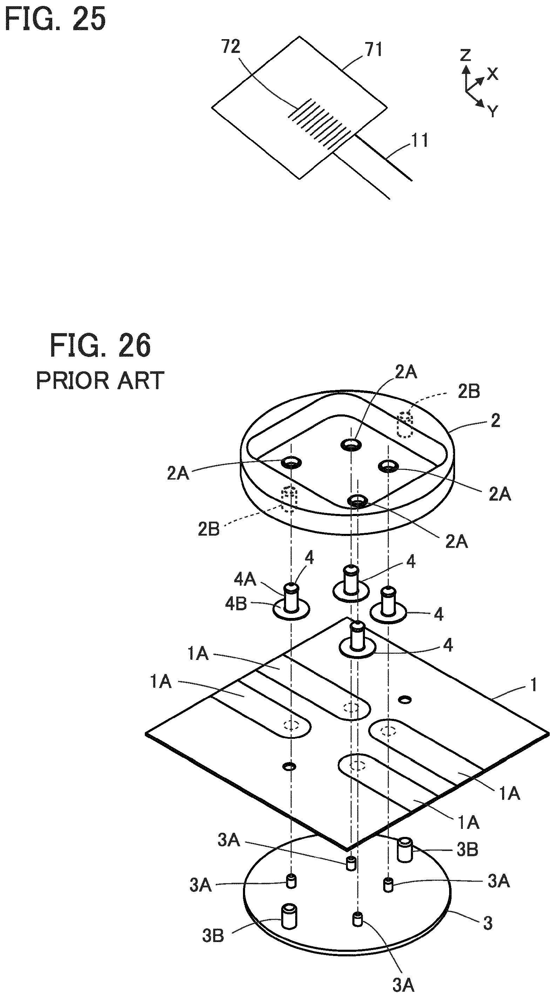

In addition, as shown in FIG. 26, the housing 2 and the base member 3 are fixed to each other by press-fitting a housing fixing post 3B, which is formed to project on the base member 3, into a post accommodating portion 2B of the housing 2.

When a wearable device is fitted with the connector disclosed in JP 2018-129244 A, the wearable device can be connected to an electrode formed of a flexible conductor.

However, in the case where the wearable device is disposed apart from a measurement position, it is necessary to constitute an electrical path from the electrode disposed at the measurement position to an attachment position of the connector, and when such an electrical path is formed of the flexible conductor, electric resistance becomes high, and cost increases.

To cope with it, in order to connect an electrode formed of a flexible conductor to a wearable device by an inexpensive electric wire with low electric resistance, the development of a small-sized connector for connecting a connection object such as an electric wire to a flexible conductor disposed on a garment is desired.

As a connector of this time, by using a connector having such a configuration that a projection of a base member is inserted into a projection accommodating portion of a contact with a flexible conductor being sandwiched therebetween as with the connector disclosed in JP 2018-129244 A, a connection object such as an electric wire is connected to the contact, whereby the flexible conductor and the connection object are electrically connected to each other.

However, in the connector of JP 2018-129244 A, since the contact 4 is present, the post accommodating portion 2B and the housing fixing post 3B that are for fixing the housing 2 and the base member 3 to each other need to be separately disposed at positions away from the contact 4, and thus it is difficult to reduce a size of the connector.

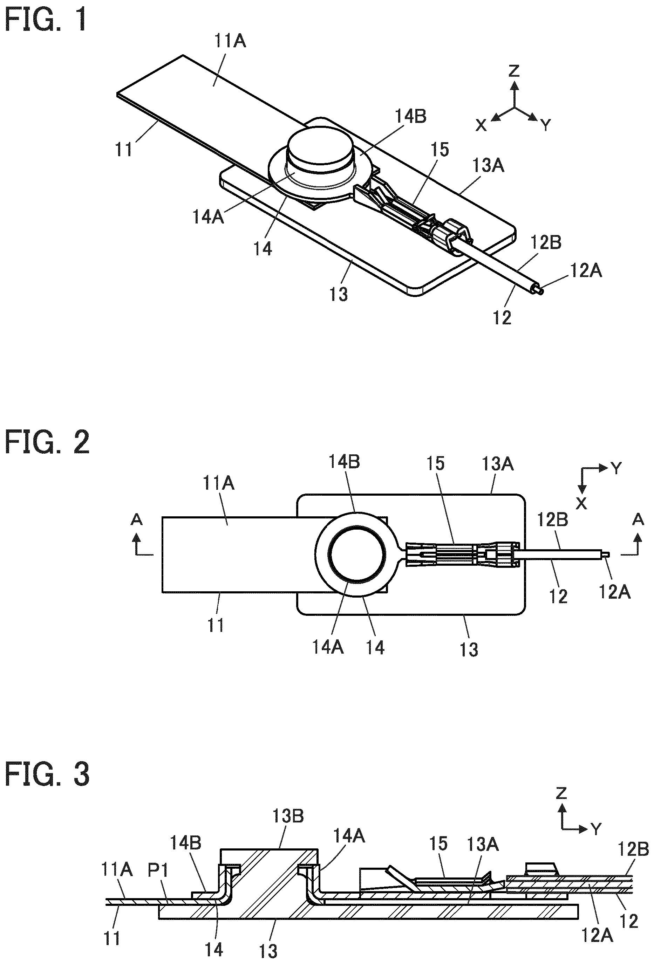

EP 3 435 490 A1 describes a first circuit board 11, a second circuit board 21 and a connection assisting member 31 which are used in a circuit board assembly, as shown in FIG. 1. The first circuit board 11 is constituted of a printed circuit board and includes a first substrate 12 having rigidity. The second circuit board 21 includes a flexible second substrate 22 having insulation properties, a second wiring portion 23 constituted of conductive layers formed on a top surface 22A and a bottom surface 22B of the flexible second substrate 22 and a plurality of H-shaped openings 24. The connection assisting member 31 includes a base plate 32 having insulation properties and a plurality of projections 33 projecting on the surface of the base plate 32. The projections 33 of the connection assisting member 31 are correspondingly fitted in through-holes 14 of the first circuit board 11. Each through-hole 14 is applied with through-hole plating, whereby a conductive first contact portion 15 is formed over the whole of an inner surface S of the through-hole 14. When the projections 33 of the connection assisting member 31 are fitted into the through-holes 14 of the first circuit board 11 through the openings 24 of the second circuit board 21, the pairs of second contact portions 26 protruding in the openings 24 of the second circuit board 21 are pushed in the +Z direction by the projections 33 and bent in the +Z direction in the through-holes 14 of the first circuit board 11. The pair of second contact portions 26 of the second circuit board 21 are sandwiched between the relevant lateral surfaces of the projection 33 and the first contact portion 15 formed on the inner surface S of the through-hole 14. The pair of second contact portions 26 of the second circuit board 21 are pressed against the first contact portion 15 of the first circuit board 11 by the projection 33. Thus, the first contact portion 15 of the first circuit board 11 and the pair of second contact portions 26 of the second circuit board 21 are electrically connected to each other in a reliable manner.

US 6 310 407 B1 describes an electrode connection structure in which an extending electrode section 5a extends from a peripheral portion of an antenna electrode 4 formed of an electrically conductive cloth portion on the surfaces of a base member 5. The extending electrode section 5a is turned back to the back surface of the base member 5 to form a triple laminate layer of the antenna electrode 4, the base member 5 and the extending electrode section 5a. The antenna electrode 4 and a connection terminal 111 are clipped electrically and mechanically by a clip member 112 in the triple laminate layer potion. The connection terminal 111 is connected to a cable 6. The connection terminal 111 comprises a first portion 112a being coupled to the antenna electrode, a second portion 112b extending from the first portion 112a and a third portion 112c arranged at the second portion 112b facing away from the first portion 112a. A diameter of the first and third portions 112a, 112c is larger than the diameter of the second portion 112b. A peripheral portion 113a of the connection terminal 111 and the antenna electrode are sandwiched between the first portion 112a and the third portion 112c of the connection terminal 111.

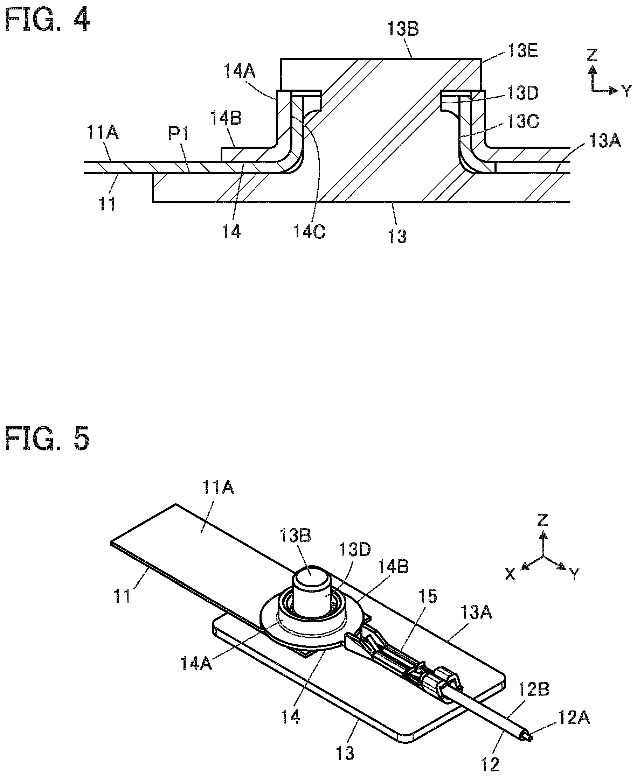

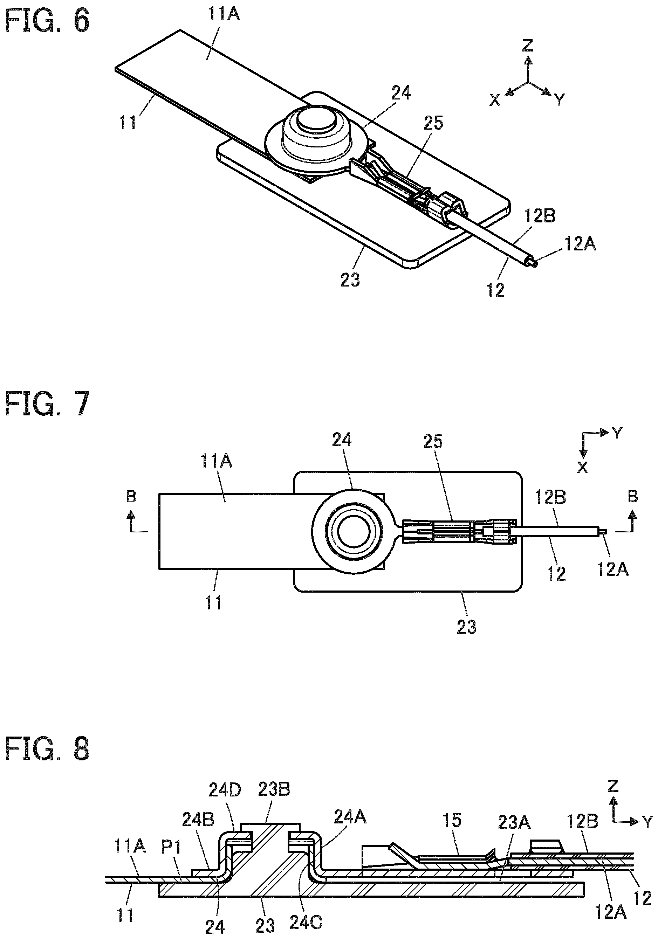

JP S56 13369 U describes a connector 5 for coupling a rigid circuit board 1 with a flexible circuit board 4, as shown in FIGS. 5 and 6. The rigid circuit board 1 has a recess, wherein an electrically conductive via 31 of the rigid circuit board 1 extends into the recess. The flexible circuit board 4 comprises an electrically insulating base layer 43, an electrically insulating cover layer 41, and an electrically conductive layer 42 sandwiched therebetween. At one end of the flexible circuit board 4, the electrically insulating cover layer 41 is removed such that a part of the electrically conductive layer 42 is exposed and coupled to the via 31 of the rigid circuit board 1. The rigid circuit board 1 is mechanically coupled to the flexible circuit board 4 by a projection of the connector 5 being inserted into the recess of the rigid circuit board 1 and a corresponding recess within the flexible circuit board 4. In this state, the electrically conductive layer 42 is sandwiched between a projection of the connector 5 and the via 31.

JP 2014 203815 A describes a connector 4 for coupling a sheet-like conductive member 2 to a plate 3, as shown in FIGS. 1 to 6. The connector 4 comprises a projection 8. Ends of the sheet-like conductive member 2 and the plate 3 are formed to be put over the projection such that the sheet-like conductive member 2 is mechanically coupled to the plate 3. The sheet-like conductive member 2 comprises electrically conductive first vias 6 and the plate 3 comprises electrically conductive second vias 7. The sheet-like conductive member 2 and the plate 3 are arranged such that the first and second vias 6, 7 touch each other.

### SUMMARY OF THE INVENTION

The present invention has been made to solve the conventional problem described above and aims at providing a connector capable of reducing the size thereof while a conductive connection object is connected to a flexible conductor of a sheet-like conductive member.

A connector according to the present invention is described by independent claim 1. Advantageous embodiments are given in the dependent claims. The connector is configured for connecting an electric wire forming a connection object having conductivity to a flexible conductor exposed at least on a top surface of a sheet-like conductive member having the top surface and a bottom surface facing in opposite directions from each other, the connector comprising:
a base member including a first surface configured for facing the bottom surface of the sheet-like conductive member and a projection formed to project on the first surface; and
a contact having conductivity, the contact including a projection accommodating portion of recess shape through which the projection of the base member penetrates and being configured for being connected to the connection object,
wherein the projection includes a root portion accommodated in the projection accommodating portion of the contact, an extending portion jointed to the root portion, and a deformed portion overhanging from a tip of the extending portion in a direction along the first surface, and
wherein the first surface of the base member is configured for making contact with the bottom surface of the sheet-like conductive member, the contact is fixed to the base member by the deformed portion of the projection in a state where the root portion of the projection is accommodated in the projection accommodating portion with the sheet-like conductive member being sandwiched therebetween, and an inner peripheral surface of the projection accommodating portion is configured for making contact with the flexible conductor of the sheet-like conductive member in a direction parallel to the first surface, whereby, in this state, the connection object is electrically connected to the flexible conductor via the contact.

### BRIEF DESCRIPTION OF THE DRAWINGS

FIG. 1 is a perspective view showing a connector according to Embodiment 1.
FIG. 2 is a plan view showing the connector according to Embodiment 1.
FIG. 3 is a cross-sectional view taken along line A-A in FIG. 2.
FIG. 4 is an enlarged view of an important part of FIG. 3.
FIG. 5 is a perspective view showing the connector according to Embodiment 1 in the process of assembling.
FIG. 6 is a perspective view showing a connector according to Embodiment 2.
FIG. 7 is a plan view showing the connector according to Embodiment 2.
FIG. 8 is a cross-sectional view taken along line B-B in FIG. 7.
FIG. 9 is an enlarged view of an important part of FIG. 8.
FIG. 10 is a perspective view showing the connector according to Embodiment 2 in the process of assembling.
FIG. 11 is a perspective view showing a connector according to Embodiment 3.
FIG. 12 is a plan view showing the connector according to Embodiment 3.
FIG. 13 is a cross-sectional view taken along line C-C in FIG. 12.
FIG. 14 is an enlarged view of an important part of FIG. 13.
FIG. 15 is a perspective view showing the connector according to Embodiment 3 in the process of assembling.
FIG. 16 is a perspective view showing a connector according to Embodiment 4.
FIG. 17 is a plan view showing the connector according to Embodiment 4.
FIG. 18 is a cross-sectional view taken along line D-D in FIG. 17.
FIG. 19 is an enlarged view of an important part of FIG. 18.
FIG. 20 is a cross-sectional view showing the connector according to Embodiment 4 in the process of assembling.
FIG. 21 is a perspective view showing a connector according to Embodiment 5.
FIG. 22 is a perspective view showing a connector according to Embodiment 6.
FIG. 23 is a plan view showing the connector according to Embodiment 6.
FIG. 24 is a perspective view showing a top surface of an electrode to which a sheet-like conductive member is connected.
FIG. 25 is a perspective view showing a bottom surface of the electrode to which the sheet-like conductive member is connected.
FIG. 26 is an exploded perspective view showing a conventional connector.
FIG. 27 is a partial cross-sectional view showing the conventional connector.

### DETAILED DESCRIPTION OF THE INVENTION

Embodiments of the present invention are described below with reference to the accompanying drawings.

### Embodiment 1

FIGS. 1 and 2 show a connector according to Embodiment 1. This connector is configured such that a covered electric wire 12 being a conductive connection object is connected to a sheet-like conductive member 11, and includes a base member 13 made of an insulating resin material, and a conductive contact 14 held by the base member 13.

The sheet-like conductive member 11 has a top surface and a bottom surface facing in opposite directions from each other and has a flexible conductor 11A exposed at least on the top surface. As the sheet-like conductive member 11, conductive cloth woven using a conductive thread such as silver can be used, for example. When such conductive cloth is used, the flexible conductor 11A is exposed not only on the top surface but also on the bottom surface of the sheet-like conductive member 11. In addition, one obtained by applying a conductive ink on a surface of cloth having no conductivity by printing or another method to form the flexible conductor 11A on the surface can also be used as the sheet-like conductive member 11. Further, a member obtained by forming the flexible conductor 11A formed of a conductive pattern on a surface of an insulating sheet body of a resin film or the like may be used as the sheet-like conductive member 11.

The sheet-like conductive member 11 shown in FIGS. 1 and 2 has a band shape extending in a predetermined direction.

The covered electric wire 12 has such a structure that an outer periphery of a conductor portion 12A is covered with a covering portion 12B made of an insulating material. With the connector according to Embodiment 1, the conductor portion 12A of the covered electric wire 12 is electrically connected to the flexible conductor 11A of the sheet-like conductive member 11.

The covered electric wire 12 shown in FIGS. 1 and 2 extends in the same direction as the direction in which the sheet-like conductive member 11 of band shape extends.

The base member 13 includes a flat plate portion 13A of rectangular shape extending in parallel to the sheet-like conductive member 11, and the sheet-like conductive member 11 and the covered electric wire 12 are electrically connected to each other on a surface of the flat plate portion 13A.

The contact 14 includes a tubular portion 14A of cylindrical shape having a central axis extending in a direction perpendicular to the flat plate portion 13A of the base member 13, and a flange 14B of annular shape joined to one end of the tubular portion 14A and extending along the surface of the flat plate portion 13A.

In addition, a crimp terminal portion 15 integrally formed with the contact 14 is joined to the flange 14B of the contact 14, and an end portion of the covered electric wire 12 is connected to the crimp terminal portion 15.

For convenience, the predetermined direction in which the sheet-like conductive member 11 extends toward the base member 13 is called "+Y direction," the width direction of the sheet-like conductive member 11 of band shape "X direction," and the direction orthogonal to an XY plane "Z direction."

The sheet-like conductive member 11 and the flat plate portion 13A of the base member 13 extend along an XY plane, a +Y directional end portion of the sheet-like conductive member 11 is connected to the contact 14, and a -Y directional end portion of the covered electric wire 12 is connected to the contact 14 via the crimp terminal portion 15.

As shown in FIG. 3, a +Z directional surface of the flat plate portion 13A of the base member 13 forms a first surface P1, and a projection 13B of substantially columnar shape extending in the +Z direction is formed to project on the first surface P1.

A cut-out portion (not shown) is formed near the +Y directional end portion of the sheet-like conductive member 11, and the projection 13B of the base member 13 penetrates the cut-out portion of the sheet-like conductive member 11 and the tubular portion 14A of the contact 14. Therefore, the sheet-like conductive member 11 is sandwiched between the tubular portion 14A of the contact 14 and an outer peripheral surface of the projection 13B.

In addition, the crimp terminal portion 15 integrally formed with the contact 14 is disposed on the surface of the flat plate portion 13A of the base member 13, and the conductor portion 12A at the -Y directional end portion of the covered electric wire 12 is drawn from the covering portion 12B and crimped on the crimp terminal portion 15.

As shown in FIG. 4 that is an enlarged partial view of FIG. 3, the projection 13B of the base member 13 includes a root portion 13C joined to the first surface P1, an extending portion 13D extending in the +Z direction from a +Z directional end portion of the root portion 13C, and a deformed portion 13E joined to a +Z directional end portion of the extending portion 13D.

In addition, a projection accommodating portion 14C of recess shape through which the projection 13B penetrates is formed inside the tubular portion 14A of the contact 14, and the root portion 13C and the extending portion 13D of the projection 13B are accommodated in the projection accommodating portion 14C.

While the first surface P1 of the base member 13 makes contact with the bottom surface on the -Z direction side of the sheet-like conductive member 11, and the flexible conductor 11A is exposed at least on the top surface on the +Z direction side of the sheet-like conductive member 11, the sheet-like conductive member 11 is bent at a right angle at a position where the projection 13B is formed, and extends in the +Z direction while being sandwiched between an outer peripheral surface of the root portion 13C of the projection 13B and an inner peripheral surface of the projection accommodating portion 14C of the contact 14. Accordingly, the inner peripheral surface of the projection accommodating portion 14C of the contact 14 comes into contact with the flexible conductor 11A of the sheet-like conductive member 11 in a direction along an XY plane.

Here, the projection accommodating portion 14C of the contact 14 is formed in advance to have an inside diameter slightly smaller than a dimension obtained by adding a double of a thickness of the sheet-like conductive member 11 to an outside diameter of the root portion 13C of the projection 13B; therefore, the flexible conductor 11A of the sheet-like conductive member 11 is pressed by the root portion 13C of the projection 13B against the inner peripheral surface of the projection accommodating portion 14C of the contact 14 such that a predetermined contact pressure is applied thereto, whereby the contact 14 is electrically connected to the flexible conductor 11A.

In addition, while the tubular portion 14A of the contact 14 has a shape whose +Z directional end portion is opened, the deformed portion 13E of the projection 13B overhangs along an XY plane from the +Z directional end portion of the extending portion 13D and covers the +Z directional end portion of the tubular portion 14A. Consequently, the contact 14 is sandwiched between the deformed portion 13E of the projection 13B and the first surface P1 of the base member 13 and fixed to the base member 13 via the sheet-like conductive member 11.

By inserting the root portion 13C of the projection 13B into the projection accommodating portion 14C of the contact 14 with the sheet-like conductive member 11 being sandwiched therebetween when the deformed portion 13E is not formed at a tip of the extending portion 13D of the projection 13B, the extending portion 13D of the projection 13B is allowed to project in the +Z direction from the tubular portion 14A of the contact 14 as shown in FIG. 5, and thereafter, the deformed portion 13E is formed through heat deformation of the +Z directional end portion of the extending portion 13D of the projection 13B, whereby the connector as above can be assembled.

Note that the conductor portion 12A at the -Y directional end portion of the covered electric wire 12 is drawn from the covering portion 12B and crimped on the crimp terminal portion 15 in advance, and accordingly, the conductor portion 12A of the covered electric wire 12 is electrically connected to the flexible conductor 11A of the sheet-like conductive member 11 via the contact 14 and the crimp terminal portion 15.

In the connector of Embodiment 1, the contact 14 is fixed to the base member 13 by means of the deformed portion 13E situated at a +Z directional end portion of the projection 13B while the flexible conductor 11A of the sheet-like conductive member 11 and the contact 14 are electrically connected to each other with the root portion 13C situated at a -Z directional end portion of the projection 13B of the base member 13. Therefore, unlike the connector of JP 2018-129244 A, it is not necessary to dispose a fixing member at a position away from the contact, whereby the connector can be decreased in size.

When the connector of Embodiment 1 is applied to a smart cloth, and an electrode (not shown) is connected to the flexible conductor 11A of the sheet-like conductive member 11, the electrode disposed at a measurement position and a wearable device can be connected to each other by means of the inexpensive covered electric wire 12 with low electric resistance.

In the connector of Embodiment 1, presence of the deformed portion 13E of the projection 13B allows the flange 14B of the contact 14 to come into contact with the flexible conductor 11A of the sheet-like conductive member 11 disposed on the first surface P1 of the base member 13 with a predetermined contact pressure, and thus the contact 14 is also electrically connected to the flexible conductor 11A via the flange 14B, whereby the reliability of electric connection between the contact 14 and the flexible conductor 11A can be improved.

### Embodiment 2

FIGS. 6 and 7 show a connector according to Embodiment 2. As with the connector of Embodiment 1, this connector is configured such that a covered electric wire 12 being a conductive connection object is connected to a sheet-like conductive member 11, and includes a base member 23 made of an insulating resin material, and a conductive contact 24 held by the base member 23.

The sheet-like conductive member 11 and the covered electric wire 12 are the same as those used in Embodiment 1.

As shown in FIG. 8, as with the base member 13 used in Embodiment 1, the base member 23 includes a flat plate portion 23A of rectangular shape extending along an XY plane, a +Z directional surface of the flat plate portion 23A forms a first surface P1, and a projection 23B extending in the +Z direction is formed to project on the first surface P1.

As with the contact 14 used in Embodiment 1, the contact 24 includes a tubular portion 24A of cylindrical shape, and a flange 24B joined to a -Z directional end portion of the tubular portion 24A. A projection accommodating portion 24C is formed inside the tubular portion 24A, and a crimp terminal portion 25 integrally formed with the contact 24 is joined to the flange 24B.

The contact 24 further includes a top plate portion 24D of annular shape joined to a +Z directional end portion of the tubular portion 24A. The top plate portion 24D extends along an XY plane toward a central axis of the tubular portion 24A so as to cover a +Z directional end portion of the projection accommodating portion 24C formed inside the tubular portion 24A.

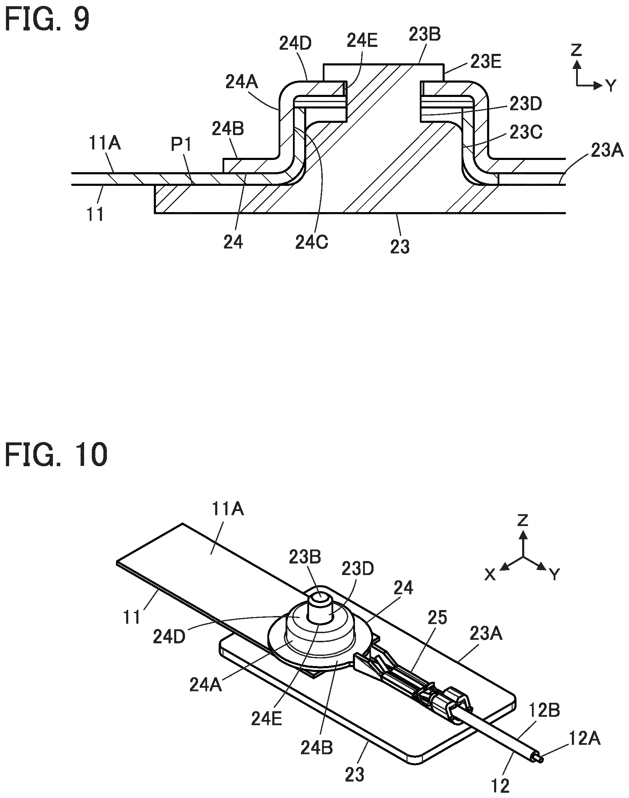

As shown in FIG. 9 that is a partial enlarged view of FIG. 8, the projection 23B of the base member 23 includes a root portion 23C joined to the first surface P1, an extending portion 23D extending in the +Z direction from a +Z directional end portion of the root portion 23C, and a deformed portion 23E joined to a +Z directional end portion of the extending portion 23D. In addition, the top plate portion 24D of the contact 24 is provided with a through hole 24E through which the extending portion 23D of the projection 23B passes.

On the +Z direction side of the top plate portion 24D of the contact 24, the deformed portion 23E of the projection 23B overhangs along an XY plane from the +Z directional end portion of the extending portion 23D and covers the top plate portion 24D around the through hole 24E. Consequently, the contact 24 is sandwiched between the deformed portion 23E of the projection 23B and the first surface P1 of the base member 23 and fixed to the base member 23 via the sheet-like conductive member 11.

As with Embodiment 1, the first surface P1 of the base member 23 makes contact with the bottom surface on the -Z direction side of the sheet-like conductive member 11, and the sheet-like conductive member 11 is bent at a right angle at a position where the projection 23B is formed, and extends in the +Z direction while being sandwiched between an outer peripheral surface of the root portion 23C of the projection 23B and an inner peripheral surface of the projection accommodating portion 24C of the contact 24. Accordingly, the inner peripheral surface of the projection accommodating portion 24C of the contact 24 comes into contact with the flexible conductor 11A of the sheet-like conductive member 11 in a direction along an XY plane.

Here, the projection accommodating portion 24C of the contact 24 is formed in advance to have an inside diameter slightly smaller than a dimension obtained by adding a double of a thickness of the sheet-like conductive member 11 to an outside diameter of the root portion 23C of the projection 23B; therefore, the flexible conductor 11A of the sheet-like conductive member 11 is pressed by the root portion 23C of the projection 23B against the inner peripheral surface of the projection accommodating portion 24C of the contact 24 such that a predetermined contact pressure is applied thereto, whereby the contact 24 is electrically connected to the flexible conductor 11A.

By inserting the root portion 23C of the projection 23B into the projection accommodating portion 24C of the contact 24 with the sheet-like conductive member 11 being sandwiched therebetween when the deformed portion 23E is not formed at a tip of the extending portion 23D of the projection 23B, the extending portion 23D of the projection 23B is allowed to project in the +Z direction from the through hole 24E of the contact 24 as shown in FIG. 10, and thereafter, the deformed portion 23E is formed through heat deformation of the +Z directional end portion of the extending portion 23D of the projection 23B, whereby the connector as above can be assembled.

Note that a conductor portion 12A at the -Y directional end portion of the covered electric wire 12 is drawn from a covering portion 12B and crimped on the crimp terminal portion 25 in advance, and accordingly, the conductor portion 12A of the covered electric wire 12 is electrically connected to the flexible conductor 11A of the sheet-like conductive member 11 via the crimp terminal portion 25 and the contact 24.

As with the connector of Embodiment 1, also in the connector of Embodiment 2, the contact 24 is fixed to the base member 23B by means of the deformed portion 23E situated at a +Z directional end portion of the projection 23B while the flexible conductor 11A of the sheet-like conductive member 11 and the contact 24 are electrically connected to each other with the root portion 23C situated at a -Z directional end portion of the projection 23B of the base member 23. Therefore, unlike the connector of JP 2018-129244 A, it is not necessary to dispose a fixing member at a position away from the contact, whereby the connector can be decreased in size.

Further, presence of the deformed portion 23E of the projection 23B allows the flange 24B of the contact 24 to come into contact with the flexible conductor 11A of the sheet-like conductive member 11 disposed on the first surface P1 of the base member 23 with a predetermined contact pressure, and thus the contact 24 is also electrically connected to the flexible conductor 11A via the flange 24B, whereby the reliability of electric connection between the contact 24 and the flexible conductor 11A can be improved.

### Embodiment 3

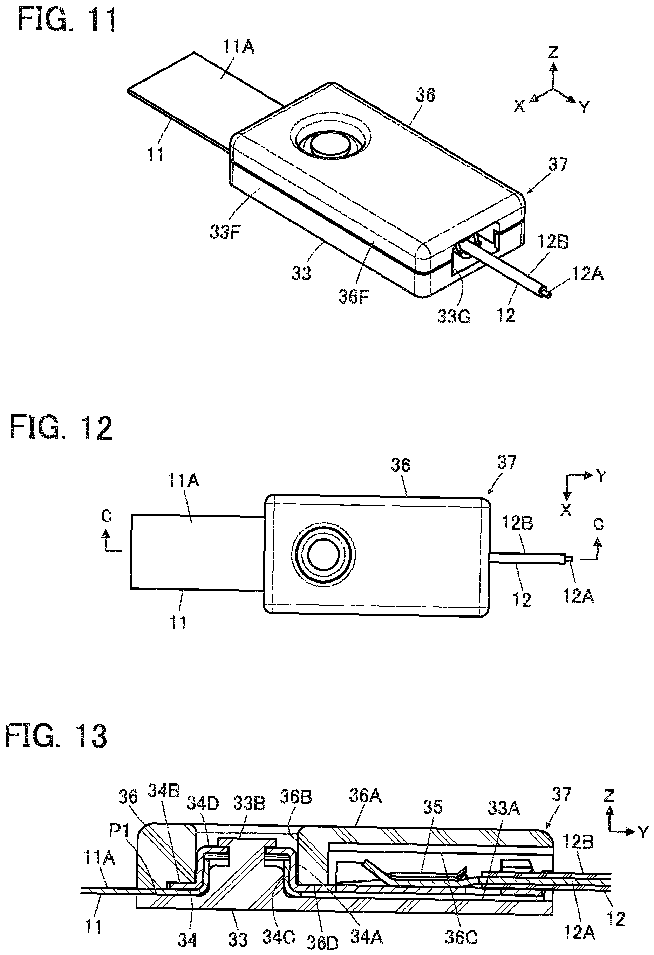

FIGS. 11 and 12 show a connector according to Embodiment 3. As with the connectors of Embodiments 1 and 2, this connector is configured such that a covered electric wire 12 being a conductive connection object is connected to a sheet-like conductive member 11, and includes a base member 33 and a top member 36 that are each made of an insulating resin material.

The sheet-like conductive member 11 and the covered electric wire 12 are the same as those used in Embodiments 1 and 2.

The base member 33 and the top member 36 have the same flat shape and the same size when viewed in the Z direction. A side wall portion 33F projecting in the +Z direction is formed at a periphery of the base member 33 while a side wall portion 36F projecting in the -Z direction is formed at a periphery of the top member 36, and these side wall portions 33F and 36F are joined to each other in the Z direction to thereby form a housing 37 of cuboidal shape composed of the base member 33 and the top member 36.

Cutout portions 33G are separately formed at a -Y directional end portion and a +Y directional end portion of the side wall portion 33F of the base member 33, and the sheet-like conductive member 11 and the covered electric wire 12 extend in the Y direction from the housing 37 separately through the cutout portions 33G.

As shown in FIG. 13, as with the base member 23 used in Embodiment 2, the base member 33 includes a flat plate portion 33A of rectangular shape extending along an XY plane, a +Z direction side surface of the flat plate portion 33A forms a first surface P1, and a projection 33B extending in the +Z direction is formed to project on the first surface P1.

A conductive contact 34 is held by the base member 33. As with the contact 24 used in Embodiment 2, the contact 34 includes a tubular portion 34A of cylindrical shape, a flange 34B joined to a -Z directional end portion of the tubular portion 34A, and a top plate portion 34D of annular shape joined to a +Z directional end portion of the tubular portion 34A. A projection accommodating portion 34C is formed inside the tubular portion 34A, and a crimp terminal portion 35 integrally formed with the contact 34 is joined to the flange 34B.

The top member 36 is joined to the base member 33 to cover the base member 33 from the +Z direction and includes a flat plate portion 36A facing the flat plate portion 33A of the base member 33. The side wall portion 36F shown in FIG. 11 is formed to project in the -Z direction from a periphery of the flat plate portion 36A. In addition, the top member 36 is provided with a recessed portion 36B at a position corresponding to the projection 33B of the base member 33, and a crimp terminal accommodating portion 36C of recess shape at a position corresponding to the crimp terminal portion 35.

The recessed portion 36B penetrates the top member 36 in the Z direction, and a flange pressing portion 36D of annular shape extending along an XY plane and facing in the -Z direction is formed at a periphery of a -Z directional end portion of the recessed portion 36B.

In addition, the crimp terminal accommodating portion 36C is formed on the -Z direction side of the flat plate portion 36A, and the crimp terminal portion 35 joined to the contact 34 is accommodated in the crimp terminal accommodating portion 36C.

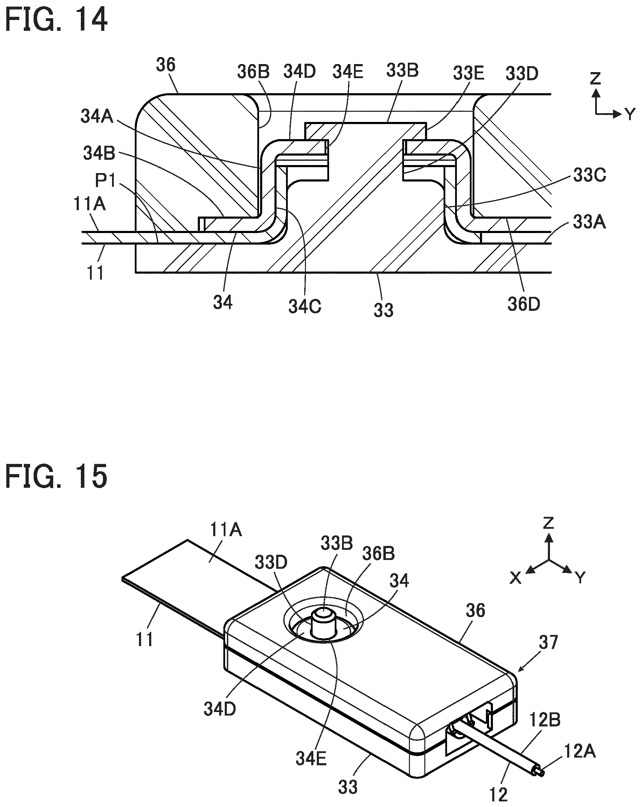

As shown in FIG. 14 that is a partial enlarged view of FIG. 13, as with the projection 23B of the base member 23 used in Embodiment 2, the projection 33B of the base member 33 includes a root portion 33C joined to the first surface P1, an extending portion 33D extending in the +Z direction from a +Z directional end portion of the root portion 33C, and a deformed portion 33E joined to a +Z directional end portion of the extending portion 33D. In addition, the top plate portion 34D of the contact 34 is provided with a through hole 34E through which the extending portion 33D of the projection 33B passes.

On the +Z direction side of the top plate portion 34D of the contact 34, the deformed portion 33E of the projection 33B overhangs along an XY plane from the +Z directional end portion of the extending portion 33D and covers the top plate portion 34D around the through hole 34E. Consequently, the contact 34 is sandwiched between the deformed portion 33E of the projection 33B and the first surface P1 of the base member 33 and fixed to the base member 33 via the sheet-like conductive member 11.

In addition, the deformed portion 33E of the projection 33B is exposed in the +Z direction from the recessed portion 36B of the top member 36.

As with Embodiments 1 and 2, the first surface P1 of the base member 33 makes contact with the bottom surface on the -Z direction side of the sheet-like conductive member 11, and the sheet-like conductive member 11 is bent at a right angle at a position where the projection 33B is formed, and extends in the +Z direction while being sandwiched between an outer peripheral surface of the root portion 33C of the projection 33B and an inner peripheral surface of the projection accommodating portion 34C of the contact 34. Accordingly, the inner peripheral surface of the projection accommodating portion 34C of the contact 34 comes into contact with the flexible conductor 11A of the sheet-like conductive member 11A in a direction along an XY plane.

Here, the projection accommodating portion 34C of the contact 34 is formed in advance to have an inside diameter slightly smaller than a dimension obtained by adding a double of a thickness of the sheet-like conductive member 11 to an outside diameter of the root portion 33C of the projection 33B; therefore, the flexible conductor 11A of the sheet-like conductive member 11 is pressed by the root portion 33C of the projection 33B against the inner peripheral surface of the projection accommodating portion 34C of the contact 34 such that a predetermined contact pressure is applied thereto, whereby the contact 34 is electrically connected to the flexible conductor 11A.

In Embodiment 3, the projection 33B of the base member 33 and the tubular portion 34A of the contact 34 are accommodated in the recessed portion 36B penetrating the top member 36 in the Z direction, and the flange pressing portion 36D of annular shape formed at the periphery of the -Z directional end portion of the recessed portion 36B makes contact with the flange 34B of the contact 34 to press the flange 34B in the -Z direction toward the flexible conductor 11A of the sheet-like conductive member 11.

By inserting the root portion 33C of the projection 33B into the projection accommodating portion 34C of the contact 34 with the sheet-like conductive member 11 being sandwiched therebetween when the deformed portion 33E is not formed at a tip of the extending portion 33D of the projection 33B, the extending portion 33D of the projection 33B is allowed to pass through the through hole 34E of the contact 34 and project in the +Z direction from the recessed portion 36B of the top member 36 as shown in FIG. 15, and thereafter, the deformed portion 33E is formed through heat deformation of the +Z directional end portion of the extending portion 33D of the projection 33B, whereby the connector as above can be assembled.

Note that a conductor portion 12A at the -Y directional end portion of the covered electric wire 12 is drawn from a covering portion 12B and crimped on the crimp terminal portion 35 in advance, and accordingly, the conductor portion 12A of the covered electric wire 12 is electrically connected to the flexible conductor 11A of the sheet-like conductive member 11 via the crimp terminal portion 35 and the contact 34.

As with the connectors of Embodiments 1 and 2, also in the connector of Embodiment 3, the contact 34 is fixed to the base member 33 by means of the deformed portion 33E situated at a +Z directional end portion of the projection 33B while the flexible conductor 11A of the sheet-like conductive member 11 and the contact 34 are electrically connected to each other with the root portion 33C situated at a -Z directional end portion of the projection 33B of the base member 33. Therefore, unlike the connector of JP 2018-129244 A, it is not necessary to dispose a fixing member at a position away from the contact, whereby the connector can be decreased in size.

Further, since not only the contact 34 is fixed to the base member 33 while being sandwiched between the deformed portion 33E of the projection 33B and the first surface P1 of the base member 33 via the sheet-like conductive member 11 but also the flange 34B of the contact 34 is pressed in the -Z direction toward the flexible conductor 11A of the sheet-like conductive member 11 by the flange pressing portion 36D of the top member 36, the contact 34 is also electrically connected to the flexible conductor 11A via the flange 34B, whereby the reliability of electric connection between the contact 34 and the flexible conductor 11A can be improved.

In addition, by applying a water-resistant adhesive between the flat plate portion 33A of the base member 33 and a -Z directional surface of the sheet-like conductive member 11, between a +Z directional surface of the sheet-like conductive member 11 and the flange 34B of the contact 34, between the flange 34B of the contact 34 and the flange pressing portion 36D of the top member 36, and between the top plate portion 34D of the contact 34 and the deformed portion 33E of the projection 33B, water can be prevented from penetrating from the outside of the connector to a connection part between the contact 34 and the flexible conductor 11A.

Further, by bonding a joint part between the side wall portion 33F of the base member 33 and the side wall portion 36F of the top member 36 with a water-resistant adhesive and sealing a drawn part of the sheet-like conductive member 11 drawn in the - Y direction from the housing 37, and a drawn part of the covered electric wire 12 drawn in the +Y direction from the housing 37, as well as the top plate portion 34D of the contact 34 and the deformed portion 33E of the projection 33B that are exposed in the +Z direction from the recessed portion 36B of the top member 36 using a water-resistant adhesive, a waterproof connector can be configured such that water is prevented from penetrating to the inside of the housing 37.

Note that, even when a contact in which a +Z directional end portion of a tubular portion is opened and which has no top plate portion like the contact 14 of Embodiment 1 is used instead of the contact 34, and a base member provided with, like the projection 13B of Embodiment 1, a projection having a deformed portion covering a +Z directional end portion of the tubular portion is used instead of the base member 33 provided with the projection 33B, a connector having the same effect as that in Embodiment 3 can be obtained.

### Embodiment 4

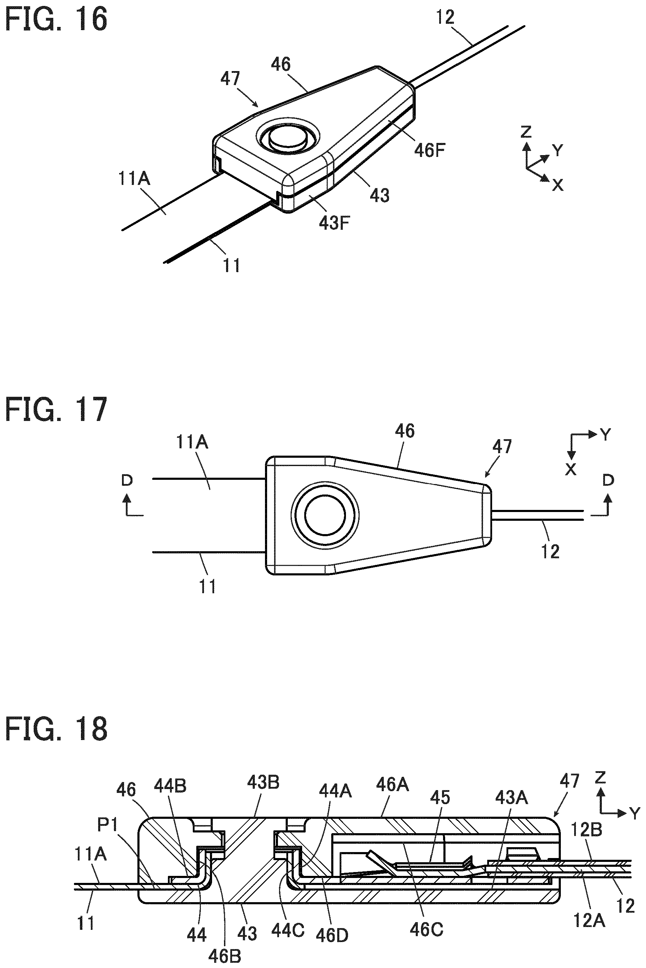

FIGS. 16 and 17 show a connector according to Embodiment 4. As with the connector of Embodiment 3, this connector is configured such that a covered electric wire 12 being a conductive connection object is connected to a sheet-like conductive member 11, and includes a base member 43 and a top member 46 each made of an insulating resin material.

The sheet-like conductive member 11 and the covered electric wire 12 are the same as those used in Embodiments 1 to 3.

The base member 43 and the top member 46 each have a flat shape apparently formed of a rectangular shape situated on the -Y direction side combined with a trapezoidal shape situated on the +Y direction side when viewed in the Z direction, and an X directional width that is narrower at its +Y directional end portion than at its -Y directional end portion. A side wall portion 43F projecting in the +Z direction is formed at a periphery of the base member 43 while a side wall portion 46F projecting in the -Z direction is formed at a periphery of the top member 46, and these side wall portions 43F and 46F are joined to each other in the Z direction to thereby form a housing 47 composed of the base member 43 and the top member 46.

As shown in FIG. 18, as with the base member 13 used in Embodiment 1, the base member 43 includes a flat plate portion 43A extending along an XY plane, a +Z directional surface of the flat plate portion 43A forms a first surface P1, and a projection 43B extending in the +Z direction is formed to project on the first surface P1.

A conductive contact 44 is held by the base member 43. As with the contact 14 used in Embodiment 1, the contact 44 includes a tubular portion 44A of cylindrical shape having a central axis along the Z direction, and a flange 44B joined to a -Z directional end portion of the tubular portion 44A. A projection accommodating portion 44C is formed inside the tubular portion 44A, and a crimp terminal portion 45 integrally formed with the contact 44 is joined to the flange 44B.

The top member 46 is joined to the base member 43 to cover the base member 43 from the +Z direction and includes a flat plate portion 46A facing the flat plate portion 43A of the base member 43. In addition, the top member 46 is provided with a contact accommodating portion 46B of recess shape opened in the -Z direction at a position corresponding to the projection 43B of the base member 43, and a crimp terminal accommodating portion 46C of recess shape at a position corresponding to the crimp terminal portion 45.

A flange pressing portion 46D of annular shape extending along an XY plane and facing in the -Z direction is formed at a periphery of a -Z directional end portion of the contact accommodating portion 46B.

In addition, the crimp terminal accommodating portion 46C is formed on the -Z direction side of the flat plate portion 43A, and the crimp terminal portion 45 joined to the contact 44 is accommodated in the crimp terminal accommodating portion 46C.

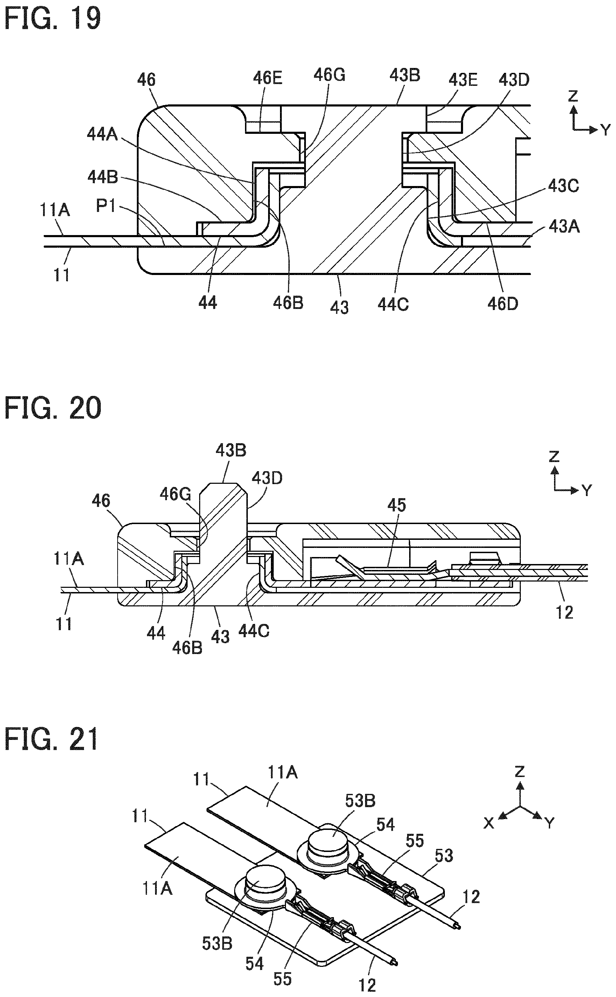

As shown in FIG. 19 that is a partial enlarged view of FIG. 18, as with the projection 13B of the base member 13 used in Embodiment 1, the projection 43B of the base member 43 includes a root portion 43C joined to the first surface P1, an extending portion 43D extending in the +Z direction from a +Z directional end portion of the root portion 43C, and a deformed portion 43E joined to a +Z directional end portion of the extending portion 43D.

The top member 46 includes an overhang portion 46E of annular shape extending along an XY plane so as to cover a +Z directional end portion of the contact accommodating portion 46B, and the overhang portion 46E is provided with an opening portion 46G through which the extending portion 43D of the projection 43B passes.

On the +Z direction side of the overhang portion 46E of the top member 46, the deformed portion 43E of the projection 43B overhangs along an XY plane from the +Z directional end portion of the extending portion 43D and covers the overhang portion 46E around the opening portion 46G. Consequently, the top member 46 is sandwiched between the deformed portion 43E of the projection 43B and the first surface P1 of the base member 43 and fixed to the base member 43 via the sheet-like conductive member 11.

Further, the flange pressing portion 46D of annular shape formed at a periphery of a -Z directional end portion of the contact accommodating portion 46B of the top member 46 presses the flange 44B of the contact 44 in the -Z direction toward the flexible conductor 11A of the sheet-like conductive member 11 so that the contact 44 is fixed to the base member 43 via the top member 46.

As with Embodiment 1, the first surface P1 of the base member 43 makes contact with the bottom surface on the -Z direction side of the sheet-like conductive member 11, and the sheet-like conductive member 11 is bent at a right angle at a position where the projection 43B is formed, and extends in the +Z direction while being sandwiched between an outer peripheral surface of the root portion 43C of the projection 43B and an inner peripheral surface of the projection accommodating portion 44C of the contact 44. Accordingly, the inner peripheral surface of the projection accommodating portion 44C of the contact 44 comes into contact with the flexible conductor 11A of the sheet-like conductive member 11 in a direction along an XY plane.

Here, the projection accommodating portion 44C of the contact 44 is formed in advance to have an inside diameter slightly smaller than a dimension obtained by adding a double of a thickness of the sheet-like conductive member 11 to an outside diameter of the root portion 43C of the projection 43B; therefore, the flexible conductor 11A of the sheet-like conductive member 11 is pressed by the root portion 43C of the projection 43B against the inner peripheral surface of the projection accommodating portion 44C of the contact 44 such that a predetermined contact pressure is applied thereto, whereby the contact 44 is electrically connected to the flexible conductor 11A.

The root portion 43C of the projection 43B is inserted into the projection accommodating portion 44C of the contact 44 with the sheet-like conductive member 11 being sandwiched therebetween when the deformed portion 43E is not formed at a tip of the extending portion 43D of the projection 43B, and the extending portion 43D of the projection 43B is allowed to pass through the opening portion 46G of the top member 46 from the projection accommodating portion 44C of the contact 44 as shown in FIG. 20, and thereafter, the deformed portion 43E is formed through heat deformation of the +Z directional end portion of the extending portion 43D of the projection 43B projecting in the +Z direction from the opening portion 46G of the top member 46, whereby the connector as above can be assembled.

Note that a conductor portion 12A at the -Y directional end portion of the covered electric wire 12 is drawn from a covering portion 12B and crimped on the crimp terminal portion 45 in advance, and accordingly, the conductor portion 12A of the covered electric wire 12 is electrically connected to the flexible conductor 11A of the sheet-like conductive member 11 via the crimp terminal portion 45 and the contact 44.

As with the connectors of Embodiments 1 to 3, also in the connector of Embodiment 4, the contact 44 is fixed to the base member 43 by fixing the top member 46 to the base member 43 by means of the deformed portion 43E situated at a +Z directional end portion of the projection 43B while the flexible conductor 11A of the sheet-like conductive member 11 and the contact 44 are electrically connected to each other with the root portion 43C situated at a -Z directional end portion of the projection 43B of the base member 43. Therefore, unlike the connector of JP 2018-129244 A, it is not necessary to dispose a fixing member at a position away from the contact, whereby the connector can be decreased in size.

Further, since the flange 44B of the contact 44 is pressed in the -Z direction toward the flexible conductor 11A of the sheet-like conductive member 11 by the flange pressing portion 46D of the top member 46, the contact 44 is also electrically connected to the flexible conductor 11A via the flange 44B, whereby the reliability of electric connection between the contact 44 and the flexible conductor 11A can be improved.

In addition, as with the connector of Embodiment 3, by applying a water-resistant adhesive between the flat plate portion 43A of the base member 43 and a -Z directional surface of the sheet-like conductive member 11, between a +Z directional surface of the sheet-like conductive member 11 and the flange 44B of the contact 44, between the flange 44B of the contact 44 and the flange pressing portion 46D of the top member 46, and between the overhang portion 46E of the top member 46 and the deformed portion 43E of the projection 43B, water can be prevented from penetrating from the outside of the connector to the connection part between the contact 44 and the flexible conductor 11A.

Further, by bonding a joint part between the side wall portion 43F of the base member 43 and the side wall portion 46F of the top member 46 with a water-resistant adhesive and sealing a drawn part of the sheet-like conductive member 11 drawn in the - Y direction from the housing 47 and a drawn part of the covered electric wire 12 drawn in the +Y direction from the housing 47 using a water-resistant adhesive, a waterproof connector can be configured such that water is prevented from penetrating to the inside of the housing 47.

Note that, even when a contact having a top plate portion joined to a +Z directional end portion of a tubular portion like the contact 24 of Embodiment 2 is used instead of the contact 44, and a base member provided with, like the projection 23B of Embodiment 2, a projection having a deformed portion covering part of the top plate portion is used instead of the base member 43 provided with the projection 43B, a connector having the same effect as that in Embodiment 4 can be obtained.

### Embodiment 5

FIG. 21 shows a connector according to Embodiment 5. This connector has a two-circuit configuration. Two projections 53B are formed to project on a base member 53, and two contacts 54 are fixed to the base member 53 separately by the two projections 53B. Each of the two contacts 54 is integrally joined to a corresponding crimp terminal portion 55.

The projection 53B and the contact 54 respectively have the same configurations as those of the projection 13B and the contact 14 used in Embodiment 1, and two covered electric wires 12 are separately electrically connected to flexible conductors 11A of two sheet-like conductive members 11 via the two contacts 54. In other words, electrical connection can be established in the two circuits with a single connector.

Note that even when the projection 23B and the contact 24 of Embodiment 2 are used instead of the projection 53B and the contact 54, a connector having a two-circuit configuration can be similarly formed. In addition, as with Embodiments 3 and 4, a connector having a two-circuit configuration provided with a housing composed of a base member and a top member can be formed.

Further, in addition to a connector having the two-circuit configuration, it is also possible to configure a connector having a multi-circuit configuration with three or more circuits using a plurality of projections formed on a common base member and a plurality of contacts held by the plurality of projections.

### Embodiment 6

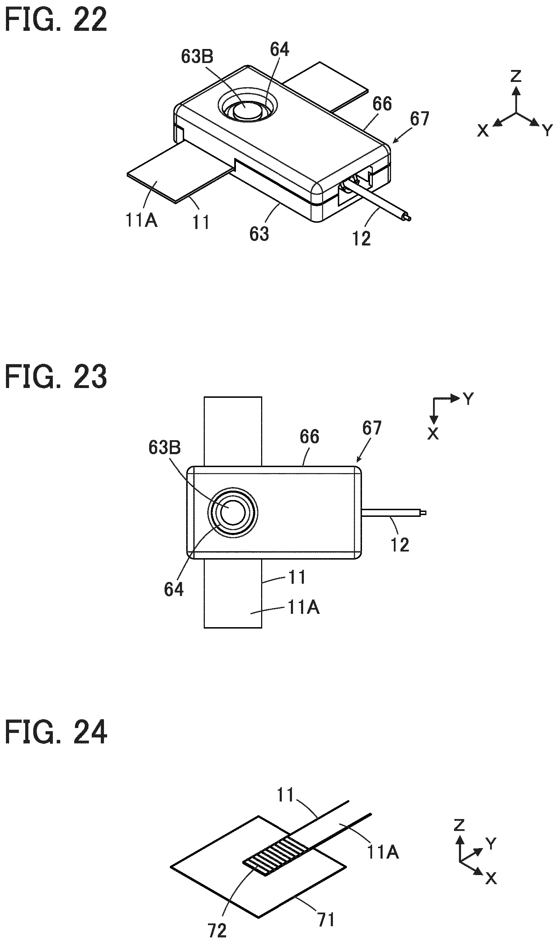

FIGS. 22 and 23 show a connector according to Embodiment 6. As with the connector of Embodiment 3, this connector includes a housing 67 of cuboidal shape composed of a base member 63 and a top member 66. The base member 63 includes a projection 63B, and a contact 64 is fixed to the base member 63 by the projection 63B. The projection 63B and the contact 64 respectively have the same configurations as those of the projection 33B and the contact 34 in Embodiment 3. A flexible conductor 11A of a sheet-like conductive member 11 is electrically connected to a covered electric wire 12 via the contact 64.

Meanwhile, not an end portion of the sheet-like conductive member 11 but a middle part of the flexible conductor 11A of the sheet-like conductive member 11 having a band shape and extending in the X direction is electrically connected to the covered electric wire 12 via the contact 64. Therefore, the sheet-like conductive member 11 extends from the housing 67 to each of opposite sides in the X direction of the housing 67.

By electrically connecting a separate electrode (not shown) to each of the flexible conductor 11A of the sheet-like conductive member 11 drawn in the -X direction from the housing 67 and the flexible conductor 11A of the sheet-like conductive member 11 drawn in the +X direction from the housing 67, the two electrodes can be electrically connected to a single covered electric wire 12. In other words, by applying the connector according to Embodiment 6 to a smart cloth, biological data obtained from the two electrodes separately disposed at two measurement positions can be sent to the single covered electric wire 12.

While the connector of Embodiment 6 has substantially the same configuration as that of the connector of Embodiment 3 shown in FIGS. 11 and 12, even when the connector has substantially the same configuration as that of the connector of Embodiment 1 shown in FIGS. 1 and 2, the sheet-like conductive member 11 connected to the single covered electric wire 12 can similarly extend in two directions, i.e., the -X direction and the +X direction. Further, even when the connector has substantially the same configuration as that of the connector of Embodiment 2 shown in FIGS. 6 and 7 or the connector of Embodiment 4 shown in FIGS. 16 and 17, the sheet-like conductive member 11 connected to the single covered electric wire 12 can similarly extend in two directions, i.e., the -X direction and the +X direction.

In addition, by making the sheet-like conductive member 11 be branched, the sheet-like conductive member 11 extending in three or more directions can be electrically connected to the single covered electric wire 12.

Note that an electrode disposed at a measurement position of a smart cloth can be formed of a conductive member having the same configuration as that of the sheet-like conductive member 11, and in this case, the sheet-like conductive member 11 integrally formed with the electrode can be connected to the covered electric wire 12 by means of any of the connectors of Embodiments 1 to 6.

In addition, when the electrode 71 and the sheet-like conductive member 11 are formed as separate components as shown in FIGS. 24 and 25, by sewing the sheet-like conductive member 11 to the electrode 71 with a conductive yarn 72 for example, the flexible conductor 11A of the sheet-like conductive member 11 is electrically connected to the electrode 71.

Note that in a case where the flexible conductor 11A of the sheet-like conductive member 11 and a conductive surface of the electrode 71 overlap and come into contact with each other, even when the sheet-like conductive member 11 is sewn to the electrode 71 with an insulating yarn, the flexible conductor 11A of the sheet-like conductive member 11 can be electrically connected to the conductive surface of the electrode 71. For example, in a case where the sheet-like conductive member 11 and the electrode 71 are each formed of a conductive fabric woven using a conductive yarn, regardless of whether a yarn used in sewing has conductivity or insulating properties, the flexible conductor 11A can be electrically connected to the electrode 71 by sewing the sheet-like conductive member 11 to the electrode 71.

In Embodiments 1 to 6 above, the covered electric wire 12 is connected to the contact 14, 24, 34, 44, 54 by press-fitting the conductor portion 12A of the covered electric wire 12 to the crimp terminal portion 15, 25, 35, 45, 55, but the invention is not limited thereto, and the covered electric wire 12 can be connected by known various methods such as screw fixing, soldering, welding, and adhesion.

In addition, in Embodiments 1 to 6 above, the covered electric wire 12 is used as a conductive connection object connected to the sheet-like conductive member 11, but an electric wire having only the conductor portion 12A whose outer periphery is not covered with the covering portion 12B made of an insulating material may also be used. Further, various conductors such as a conductor of band shape other than an electric wire can be connected to the contact 14, 24, 34, 44, 54 as a connection object.

## Claims

1. A connector for connecting an electric wire forming a connection object (12) having conductivity to a flexible conductor (11A) exposed at least on a top surface of a sheet-like conductive member (11) having the top surface and a bottom surface facing in opposite directions from each other, the connector comprising:
a base member (13, 23, 33, 43, 53, 63) including a first surface (P1) configured for facing the bottom surface of the sheet-like conductive member and a projection (13B, 23B, 33B, 43B, 53B, 63B) formed to project on the first surface; and
a contact (14, 24, 34, 44, 54, 64) having conductivity, the contact including a projection accommodating portion (14C, 24C, 34C, 44C) of recess shape through which the projection of the base member penetrates and being configured for being connected to the connection object,
wherein the projection includes a root portion (13C, 23C, 33C, 43C) accommodated in the projection accommodating portion of the contact, an extending portion (13D, 23D, 33D, 43D) jointed to the root portion, and a deformed portion (13E, 23E, 33E, 43E) overhanging from a tip of the extending portion in a direction along the first surface, and
wherein the first surface of the base member is configured for making contact with the bottom surface of the sheet-like conductive member, the contact is fixed to the base member by the deformed portion of the projection in a state where the root portion of the projection is accommodated in the projection accommodating portion with the sheet-like conductive member being sandwiched therebetween, and an inner peripheral surface of the projection accommodating portion is configured for making contact with the flexible conductor of the sheet-like conductive member in a direction parallel to the first surface, whereby, in this state, the connection object (12) is electrically connected to the flexible conductor (11A) via the contact (14, 24, 34, 44, 54, 64),
wherein the contact (14, 24, 34, 44, 54, 64) includes a tubular portion (14A, 24A, 34A, 44A) having an end facing the first surface of the base member and another end facing in an opposite direction from the first surface of the base member, and the projection accommodating portion (14C, 24C, 34C, 44C) is formed inside the tubular portion,
wherein the contact (14, 24, 34, 44) includes a flange (14B, 24B, 34B, 44B) joined to the one end of the tubular portion (14A, 24A, 34A, 44A) and extending in a direction along the first surface (P1) of the base member, and
wherein the flange is configured for making contact with the flexible conductor (11A) of the sheet-like conductive member (11).

2. The connector according to claim 1,
wherein the other end of the tubular portion (14A) is opened, and
wherein the deformed portion (13E) of the projection (13) covers the other end of the tubular portion.

3. The connector according to claim 1,
wherein the contact (24, 34) includes a top plate portion (24D, 34D) of annular shape joined to the other end of the tubular portion (24A, 34A) and provided with a through hole (24E, 34E) through which the extending portion (23D, 33D) of the projection passes, and
wherein the deformed portion (24E, 34E) of the projection covers the top plate portion around the through hole.

4. The connector according to any one of claims 1-3, comprising a top member (36) that covers the base member (33) and is fixed to the base member,
wherein the top member has a recessed portion (36B) accommodating the projection (33B) of the base member and the tubular portion (34A) of the contact (34).

5. The connector according to claim 1, comprising a top member (46) that covers the base member (43) and is fixed to the base member,
wherein the top member includes a contact accommodating portion (46B) of recess shape for accommodating the tubular portion (44A) of the contact (44), and an overhang portion (46E) of annular shape facing the contact accommodating portion and provided with an opening portion (46G) through which the extending portion (43D) of the projection (43B) passes,
wherein the deformed portion (43E) of the projection covers the overhang portion around the opening portion, and
wherein the contact is fixed to the base member via the top member.

6. The connector according to claim 1, further comprising a crimp terminal portion (15, 25, 35, 45, 55) joined to the flange (14B, 24B, 34B, 44B) and being configured to be connected with the connection object (12).

7. The connector according to any one of claims 1-6, wherein the base member (13, 23, 33, 43, 53, 63) is made of an insulating resin material.

8. The connector according to any one of claims 1-7,
wherein a plurality of the contacts (54) are fixed to the base member (53), and
wherein a plurality of the connection objects (12) are electrically connectable to a plurality of the flexible conductors (11A) via the plurality of the contacts.

## Patentansprüche

1. Verbinder zum Verbinden eines elektrischen Drahtes, der ein Verbindungsobjekt (12) mit Leitfähigkeit bildet, mit einem flexiblen Leiter (11A), der mindestens an einer oberen Fläche eines plattenartigen leitfähigen Elements (11) freiliegt, das die obere Fläche und eine untere Fläche aufweist, die in zueinander entgegengesetzte Richtungen gewandt sind, wobei der Verbinder umfasst:
ein Basiselement (13, 23, 33, 43, 53, 63), das eine erste Fläche (P1), die eingerichtet ist, um der unteren Fläche des plattenartigen leitfähigen Elements zugewandt zu sein, und einen Vorsprung (13B, 23B, 33B, 43B, 53B, 63B) aufweist, der gebildet ist, um an der ersten Fläche vorzustehen; und
einen Kontakt (14, 24, 34, 44, 54, 64) mit Leitfähigkeit, wobei der Kontakt einen Vorsprung-Aufnahmeabschnitt (14C, 24C, 34C, 44C) mit einer Aussparungsform aufweist, den der Vorsprung des Basiselements durchdringt, und der eingerichtet ist, um mit dem Verbindungsobjekt verbunden zu sein,
wobei der Vorsprung einen Wurzelabschnitt (13C, 23C, 33C, 43C), der in dem Vorsprung-Aufnahmeabschnitt des Kontakts aufgenommen ist, einen Erstreckungsabschnitt (13D, 23D, 33D, 43D), der an den Wurzelabschnitt angefügt ist, und einen verformten Abschnitt (13E, 23E, 33E, 43E), der von einer Spitze des Erstreckungsabschnitts in einer Richtung entlang der ersten Fläche überhängt, aufweist, und
wobei die erste Fläche des Basiselements eingerichtet ist, um mit der unteren Fläche des plattenartigen leitfähigen Elements in Kontakt zu kommen, der Kontakt durch den verformten Abschnitt des Vorsprungs in einem Zustand, in dem der Wurzelabschnitt des Vorsprungs in dem Vorsprung-Aufnahmeabschnitt aufgenommen ist, wobei das plattenartige leitfähige Element dazwischen sandwichartig eingeschlossen ist, an dem Basiselement befestigt ist, und eine Innenumfangsfläche des Vorsprung-Aufnahmeabschnitts eingerichtet ist, um mit dem flexiblen Leiter des plattenartigen leitfähigen Elements in einer Richtung parallel zu der ersten Fläche in Kontakt zu kommen, wodurch in diesem Zustand das Verbindungsobjekt (12) über den Kontakt (14, 24, 34, 44, 54, 64) elektrisch mit dem flexiblen Leiter (11A) verbunden ist,
wobei der Kontakt (14, 24, 34, 44, 54, 64) einen röhrenförmigen Abschnitt (14A, 24A, 34A, 44A) aufweist, dessen eines Ende der ersten Fläche des Basiselements zugewandt und dessen anderes Ende in eine von der ersten Fläche des Basiselements entgegengesetzten Richtung gewandt ist, und der Vorsprung-Aufnahmeabschnitt (14C, 24C, 34C, 44C) im Inneren des röhrenförmigen Abschnitts gebildet ist, wobei der Kontakt (14, 24, 34, 44) einen Flansch (14B, 24B, 34B, 44B) aufweist, der an das eine Ende des röhrenförmigen Abschnitts (14A, 24A, 34A, 44A) angefügt ist und sich in einer Richtung entlang der ersten Fläche (P1) des Basiselements erstreckt, und
wobei der Flansch eingerichtet ist, um mit dem flexiblen Leiter (11 A) des plattenartigen leitfähigen Elements (11) in Kontakt zu kommen.

2. Verbinder nach Anspruch 1,
wobei das andere Ende des röhrenförmigen Abschnitts (14A) geöffnet ist, und
wobei der verformte Abschnitt (13E) des Vorsprungs (13) das andere Ende des röhrenförmigen Abschnitts abdeckt.

3. Verbinder nach Anspruch 1,
wobei der Kontakt (24, 34) einen oberen Plattenabschnitt (24D, 34D) mit Ringform aufweist, der an das andere Ende des röhrenförmigen Abschnitts (24A, 34A) angefügt ist und mit einem Durchgangsloch (24E, 34E) versehen ist, durch das der Erstreckungsabschnitt (23D, 33D) des Vorsprungs verläuft, und
wobei der verformte Abschnitt (24E, 34E) des Vorsprungs den oberen Plattenabschnitt um das Durchgangsloch herum abdeckt.

4. Verbinder nach einem der Ansprüche 1 bis 3, umfassend ein oberes Element (36), das das Basiselement (33) abdeckt und an dem Basiselement befestigt ist,
wobei das obere Element einen ausgesparten Abschnitt (36B) aufweist, der den Vorsprung (33B) des Basiselements und den röhrenförmigen Abschnitt (34A) des Kontakts (34) aufnimmt.

5. Verbinder nach Anspruch 1, umfassend ein oberes Element (46), das das Basiselement (43) abdeckt und an dem Basiselement befestigt ist,
wobei das obere Element einen Kontakt-Aufnahmeabschnitt (46B) mit einer Aussparungsform zum Aufnehmen des röhrenförmigen Abschnitts (44A) des Kontakts (44) und einen Überhangabschnitt (46E) mit einer Ringform aufweist, der dem Kontakt-Aufnahmeabschnitt zugewandt ist und mit einem Öffnungsabschnitt (46G) versehen ist, durch den der Erstreckungsabschnitt (43D) des Vorsprungs (43B) verläuft, wobei der verformte Abschnitt (43E) des Vorsprungs den Überhangabschnitt um den Öffnungsabschnitt herum abdeckt, und
wobei der Kontakt über das obere Element an dem Basiselement befestigt ist.

6. Verbinder nach Anspruch 1, ferner umfassend einen Crimpanschlussabschnitt (15, 25, 35, 45, 55), der an den Flansch (14B, 24B, 34B, 44B) angefügt und eingerichtet ist, um mit dem Verbindungsobjekt (12) verbunden zu sein.

7. Verbinder nach einem der Ansprüche 1 bis 6, wobei das Basiselement (13, 23, 33, 43, 53, 63) aus einem isolierenden Harzmaterial besteht.

8. Verbinder nach einem der Ansprüche 1 bis 7,
wobei mehrere der Kontakte (54) an dem Basiselement (53) befestigt sind, und
wobei mehrere der Verbindungsobjekte (12) über die mehreren Kontakte mit mehreren der flexiblen Leiter (11 A) elektrisch verbindbar sind.

## Revendications

1. Connecteur destiné à connecter un fil électrique formant un objet de connexion (12) ayant une conductivité à un conducteur souple (11A) exposé au moins sur une surface supérieure d'un élément conducteur de type feuille (11) comportant la surface supérieure et une surface inférieure dirigées dans des directions opposées l'une à l'autre, le connecteur comprenant :
un élément de base (13, 23, 33, 43, 53, 63) comportant une première surface (P1) configurée pour être dirigée vers la surface inférieure de l'élément conducteur de type feuille et une saillie (13B, 23B, 33B, 43B, 53B, 63B) formée pour faire saillie sur la première surface ; et
un contact (14, 24, 34, 44, 54, 64) ayant une conductivité, le contact comportant une partie de réception de saillie (14C, 24C, 34C, 44C) en forme d'évidement à travers laquelle la saillie de l'élément de base pénètre et étant configurée pour être connectée à l'objet de connexion,
dans lequel la saillie comporte une partie de racine (13C, 23C, 33C, 43C) reçue dans la partie de réception de saillie du contact, une partie d'extension (13D, 23D, 33D, 43D) jointe à la partie de racine, et une partie déformée (13E, 23E, 33E, 43E) en porte-à-faux par rapport à un bout de la partie d'extension dans une direction le long de la première surface, et
dans lequel la première surface de l'élément de base est configurée pour entre en contact avec la surface inférieure de l'élément conducteur de type feuille, le contact est fixé à l'élément de base par la partie déformée de la saillie dans un état où la partie de racine de la saillie est reçue dans la partie de réception de saillie avec l'élément conducteur de type feuille étant pris en sandwich entre celles-ci, et une surface périphérique intérieure de la partie de réception de saillie est configurée pour entre en contact avec le conducteur souple de l'élément conducteur de type feuille dans une direction parallèle à la première surface, dans cet état, l'objet de connexion (12) étant ainsi électriquement connecté au conducteur souple (11A) via le contact (14, 24, 34, 44, 54, 64),
dans lequel le contact (14, 24, 34, 44, 54, 64) comporte une partie tubulaire (14A, 24A, 34A, 44A) ayant une extrémité dirigée vers la première surface de l'élément de base et une autre extrémité dirigée dans une direction opposée à la première surface de l'élément de base, et la partie de réception de saillie (14C, 24C, 34C, 44C) est formée à l'intérieur de la partie tubulaire,
dans lequel le contact (14, 24, 34, 44) comporte une bride (14B, 24B, 34B, 44B) jointe à l'extrémité de la partie tubulaire (14A, 24A, 34A, 44A) et s'étendant dans une direction le long de la première surface (P1) de l'élément de base, et
dans lequel la bride est configurée pour entrer en contact avec le conducteur souple (11A) de l'élément conducteur de type feuille (11).

2. Connecteur selon la revendication 1,
dans lequel l'autre extrémité de la partie tubulaire (14A) est ouverte, et
dans lequel la partie déformée (13E) de la saillie (13) recouvre l'autre extrémité de la partie tubulaire.

3. Connecteur selon la revendication 1,
dans lequel le contact (24, 34) comporte une partie de plaque supérieure (24D, 34D) de forme annulaire reliée à l'autre extrémité de la partie tubulaire (24A, 34A) et pourvue d'un trou traversant (24E, 34E) à travers lequel passe la partie d'extension (23D, 33D) de la saillie, et
dans lequel la partie déformée (24E, 34E) de la saillie recouvre la partie de plaque supérieure autour du trou traversant.

4. Connecteur selon l'une quelconque des revendications 1 à 3, comprenant un élément supérieur (36) qui recouvre l'élément de base (33) et est fixé à l'élément de base, dans lequel l'élément supérieur a une partie évidée (36B) recevant la saillie (33B) de l'élément de base et la partie tubulaire (34A) du contact (34).

5. Connecteur selon la revendication 1, comprenant un élément supérieur (46) qui recouvre l'élément de base (43) et est fixé à l'élément de base,
dans lequel l'élément supérieur comporte une partie de réception de contact (46B) en forme d'évidement pour recevoir la partie tubulaire (44A) du contact (44), et une partie en porte-à-faux (46E) de forme annulaire dirigée vers la partie de réception de contact et pourvue d'une partie d'ouverture (46G) à travers laquelle passe la partie d'extension (43D) de la saillie (43B),
dans lequel la partie déformée (43E) de la saillie recouvre la partie en porte-à-faux autour de la partie d'ouverture, et
dans lequel le contact est fixé à l'élément de base via l'élément supérieur.

6. Connecteur selon la revendication 1, comprenant en outre une partie de borne à sertir (15, 25, 35, 45, 55) jointe à la bride (14B, 24B, 34B, 44B) et étant configurée pour être connectée à l'objet de connexion (12).

7. Connecteur selon l'une quelconque des revendications 1 à 6, dans lequel l'élément de base (13, 23, 33, 43, 53, 63) est constitué d'un matériau en résine isolant.

8. Connecteur selon l'une quelconque des revendications 1 à 7,
dans lequel plusieurs des contacts (54) sont fixés à l'élément de base (53), et
dans lequel plusieurs des objets de connexion (12) peuvent être électriquement connectés à plusieurs des conducteurs souples (11A) via les plusieurs contacts.
